(19) Europäisches Patentamt
European Patent Office
Office européen des brevets

(11) Veröffentlichungsnummer: **0 238 969**
**A1**

(12) **EUROPÄISCHE PATENTANMELDUNG**

(21) Anmeldenummer: **87103797.4**

(22) Anmeldetag: **16.03.87**

(51) Int. Cl.⁴: **C07C 121/46**, C08G 18/00, C08G 63/00, C08G 69/00, C07C 87/34, C07C 119/042, C07C 127/15

(30) Priorität: **25.03.86 DE 3609982**

(43) Veröffentlichungstag der Anmeldung: **30.09.87 Patentblatt 87/40**

(84) Benannte Vertragsstaaten: **CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Haas, Peter, Dr.**
**Zwengenberger Strasse 43**
**D-5657 Haan 1(DE)**

(54) **Cyclobutan-diester-dinitrile, Verfahren zu ihrer Herstellung und ihre Verwendung.**

(57) Die neuen Cyclobutan-diester-dinitrile der Formel

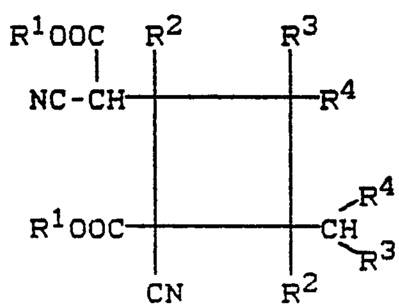

(I),

in der
$R^1$ für gegebenenfalls substituiertes Alkyl, Alkenyl, Cycloalkyl, Cycloalkenyl, Aryl, Aralkyl, Hetero(cyclo)alkyl oder Heteroaryl steht,
$R^2$, $R^3$ und $R^4$ unabhängig voneinander Wasserstoff oder gegebenenfalls substituiertes Alkyl, Aryl oder Aralkyl bedeuten und worin weiterhin
$R^2$ und $R^3$ oder $R^3$ und $R^4$ gemeinsam mit den C-Atomen, die sie substituieren, gegebenenfalls substituiertes Cycloalkyl oder Cycloalkenyl bilden können, die Sauerstoff-oder Schwefelatome als Ringglieder enthalten können,
können durch Cyclisierung von substituierten α-Cyanacrylsäureestern der Formel

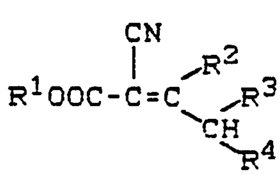

(II),

in der
$R^1$ -$R^4$ die angegebene Bedeutung besitzen,

EP 0 238 969 A1

Xerox Copy Centre

in Gegenwart von Basen erhalten werden.

Die neuen polyfunktionellen Verbindungen sind wertvolle Zwischenprodukte; sie können beispielsweise zu Polyamiden oder Polyestern polykondensiert werden.

# Cyclobutan-diester-dinitrile, Verfahren zu ihrer Herstellung und ihre Verwendung

Die Erfindung betrifft neue 1-Cyan-1-carboxyorganyl-2-($\alpha$-cyano-$\alpha$-carboxyorganyl)-methyl-cyclobutane (Cyclobutan-diester-dinitrile), ein Verfahren zu ihrer Herstellung und ihre Verwendung.

Es wurden die neuen Cyclobutan-diester-dinitrile der Formel

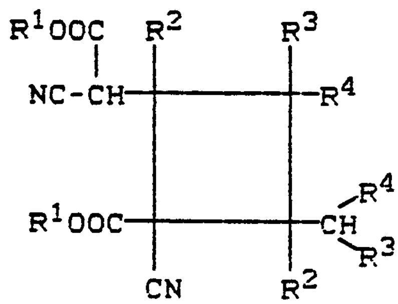

(I)

gefunden, in der

$R^1$ für gegebenenfalls substituiertes Alkyl, Alkenyl, Cycloalkyl, Cycloalkenyl, Aryl, Aralkyl, Hetero(cyclo)alkyl oder Heteroaryl steht,

$R^2$, $R^3$ und $R^4$ unabhängig voneinander Wasserstoff oder gegebenenfalls substituiertes Alkyl, Aryl, Aralkyl, Alkoxy, Aryloxy oder Aralkoxy bedeuten und worin weiterhin

$R^2$ und $R^3$ oder $R^3$ und $R^4$ gemeinsam mit den C-Atomen, die sie substituieren, Cycloalkyl oder Cycloalkenyl bilden können, die Sauerstoff-oder Schwefelatome als Ringglieder enthalten können.

Als Alkyl seien beispielsweise geradkettige oder verzweigte $C_1$-$C_{12}$-Kohlenwasserstoffreste genannt, wie Methyl, Ethyl, Propyl, Isopropyl, Butyl, Isobutyl, Hexyl, Octyl, Decyl oder Dodecyl, bevorzugt $C_1$-$C_4$-Reste, besonders bevorzugt Methyl oder Ethyl.

Als Alkenyl seien beispielsweise geradkettige oder verzweigte $C_2$-$C_{12}$-Kohlenwasserstoffreste mit einer oder mehreren olefinischen Doppelbindung(en) genannt, wie Vinyl, Propenyl, Allyl, Butenyl, Hexenyl, Octenyl oder Dodecenyl.

Als Cycloalkyl bzw. Cycloalkenyl seien beispielsweise cyclische Kohlenwasserstoffreste mit 3 - 8 C-Atomen genannt, die eine oder mehrere olefinische Doppelbindung(en) enthalten können, wie Cyclopropyl, Cyclopropenyl, Cyclobutyl, Cyclobutenyl, Cyclopentyl, Cyclopentenyl, Cyclohexyl, Cyclohexenyl, Cyclohexadienyl, Cyclooctyl, Cyclooctenyl oder Cyclooctadienyl. In bevorzugter Weise seien cyclische Reste der genannten Art mit 3 - 6, besonders bevorzugt 5 -6 C-Atomen genannt.

Als Aryl seien beispielsweise Phenyl, 1-Naphthyl oder 2-Naphthyl, in bevorzugter Weise Phenyl, genannt.

Als Aralkyl seien beispielsweise Benzyl, $\alpha$-Phenyl-ethyl, $\beta$-Phenyl-ethyl, Naphthyl-methylreste oder Naphthylethylreste, bevorzugt Benzyl genannt.

Als Hetero(cyclo)alkyl seien Reste genannt, die sich von den genannten Alkyl-, Alkenyl-, Cycloalkyl-oder Cycloalkenylresten dadurch ableiten, daß sie als Ringglieder ein oder mehrere Sauerstoff-oder Schwefelatome enthalten. Für den Fall der cyclischen Heteroalkylreste können diese über eine Methylen-oder Ethylenbrücke mit dem Ester-Sauerstoffatom verbunden sein und somit allgemein Heteroalkyl(alkyl) darstellen. Beispiele hierfür sind: Glycidyl, Oxycyclopropyl, Oxacyclobutyl, Oxacyclopentyl, Oxacyclohexyl, Oxacylobutenyl, Oxacyclopentenyl, Oxacyclohexadienyl, Thienyl, Thenyl, Tetrahydrothienyl.

Als Heteroaryl seien 5-bis 6-gliedrige aromatische Ringsysteme verstanden, die ein oder mehrere Heteroatome, wie Stickstoff-, Sauerstoff-oder Schwefelatome enthalten und aromatischen Charakter aufweisen; solche Heteroarylreste können anellierte Benzolringe tragen. Beispiele hierfür sind: Thienyl, Thenyl, Thionaphthyl, Benzofuranyl, Dibenzothiophenyl, Dibenzofuranyl, Chromanyl, Flavanyl, Thianthrenyl.

Die obengenannten Reste können ein-oder mehrfach substituiert sein. Als Substituenten hierfür seien beispielsweise genannt: Halogen, wie Fluor, Chlor, Brom oder Iod, besonders Fluor, Chlor oder Brom; $C_1$-$C_4$-Alkyl der obengenannten Art; $C_1$-$C_4$-Alkoxy, worin der $C_1$-$C_4$-Alkylrest der obengenannten Art entspricht; Estergruppen, deren Organylkomponente beispielsweise $C_1$-$C_4$-Alkyl der obengenannten Art ist; Cyano; Nitro.

Der Rest $R^1$ ist der Organylrest einer großen Anzahl verschiedenartiger Alkohole oder Phenole. Als Beispiele für solches Organyl im Rahmen der obigen allgemeinen Offenbarung seien genannt: Methyl, Ethyl, Butyl, Allyl, Glycidyl, Cyclohexyl, Cyclohexenyl, Cyclopentyl, Cyclopentenyl, Benzyl, 2-Phenylethyl, 2-Phenylethenyl, Phenyl-ethinyl, Cyanethyl, mit $C_1$-$C_4$-Alkoholen verestertes Carboxyethyl oder Carboxycarbonylethyl, Chlormethyl, Dichlormethyl, Trichlormethyl, Fluormethyl, Difluormethyl, Trifluormethyl, Chlorethyl, Fluorethyl, Tetrahydrofurfuryl, Ethyldimidazolyl, Ethyltriazolyl, Phenoxyethyl, Propargyl, Methoxyethyl, Ethenyl, 2-(2-Ethoxy-ethoxy)-ethyl, Trimethylol-propyl-bis-allylether, Trimethylolpropyl-bis-glycidylether, Hydroxyethyl, Hydroxypropyl.

In bevorzugter Weise seien Cyclobutan-diester-dinitrile genannt, in denen an die Stelle von $R^2$, $R^3$ und $R^4$ die Reste $R^{12}$, $R^{13}$ und $R^{14}$ treten, die unabhängig voneinander Wasserstoff, $C_1$-$C_{12}$-Alkyl, Phenyl oder Benzyl bedeuten.

In besonders bevorzugter Weise seien Cyclobutan-diester-dinitrile genannt, in denen an die Stelle von $R^2$, $R^3$ und $R^4$ die Reste $R^{22}$, $R^{23}$ und $R^{24}$ treten, die unabhängig voneinander Wasserstoff oder $C_1$-$C_4$-Alkyl bedeuten.

In ganz besonders bevorzugter Weise seien Cyclobutan-diester-dinitrile genannt, in denen an die Stelle von $R^2$, $R^3$ und $R^4$ die Reste $R^{32}$, $R^{33}$ und $R^{34}$ treten, wobei $R^{32}$ Wasserstoff und $R^{33}$ und $R^{34}$ unabhängig voneinander $C_1$-$C_4$-Alkyl bedeuten.

Weiterhin seien in bevorzugter Weise Cyclobutan-diester-dinitrile genannt, in denen an die Stelle von $R^1$ der Rest $R^{11}$ tritt, der $C_1$-$C_{12}$-Alkyl, $C_2$-$C_{12}$-Alkenyl, $C_5$-$C_6$-Cycloalkyl, $C_5$-$C_6$-Cycloalkenyl, Aryl, Aralkyl oder Heterocycloalkyl(alkyl) bedeutet.

In weiterer besonders bevorzugter Weise seien Cyclobutan-diester-dinitrile genannt, in denen anstelle von $R^1$ der Rest $R^{21}$ tritt, der $C_1$-$C_4$-Alkyl, $C_2$-$C_4$-Alkenyl, Phenyl, Benzyl, Phenyl-ethyl oder Glycidyl bedeutet.

Als Vertreter der neuen Cyclobutan-diester-dinitrile seien formelmäßig die folgenden aufgeführt:

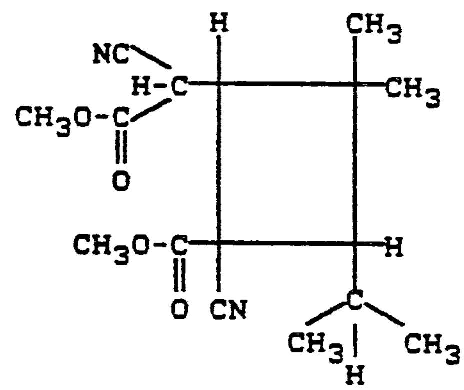

(Ia)

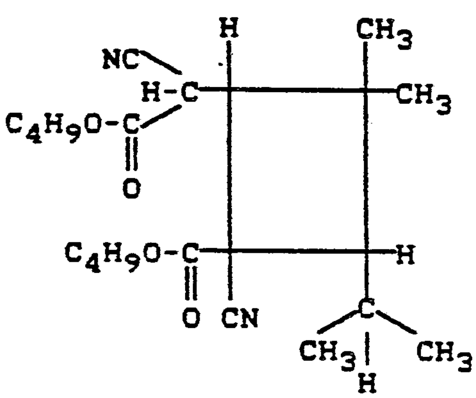

(Ib)

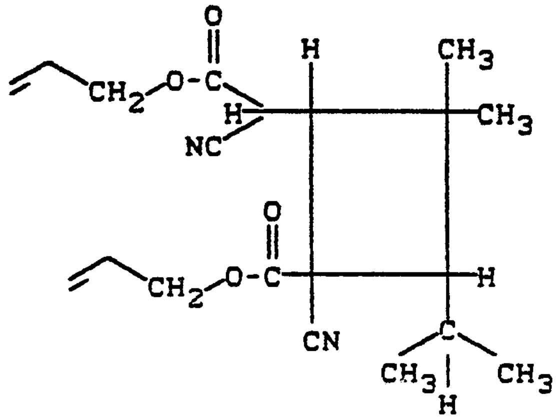

(Ic)

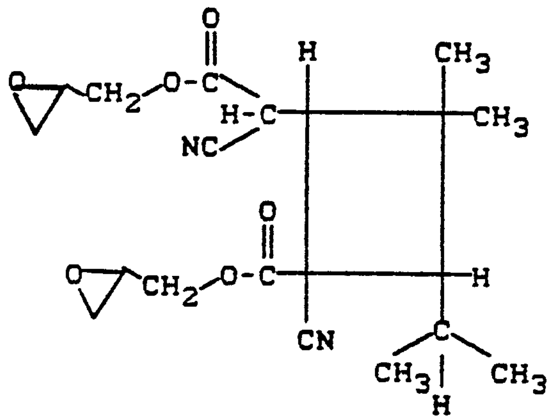

(Id)

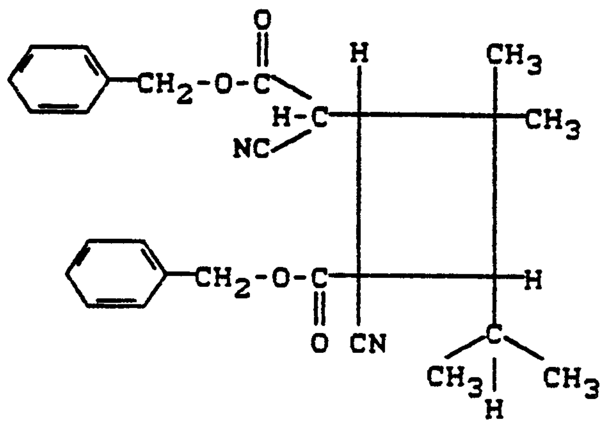

(Ie)

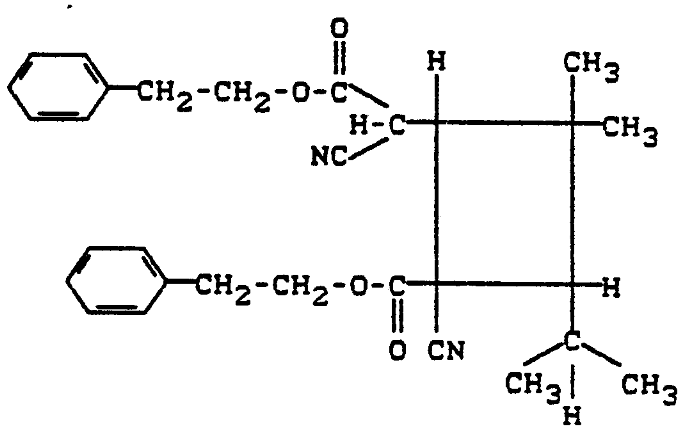

(If)

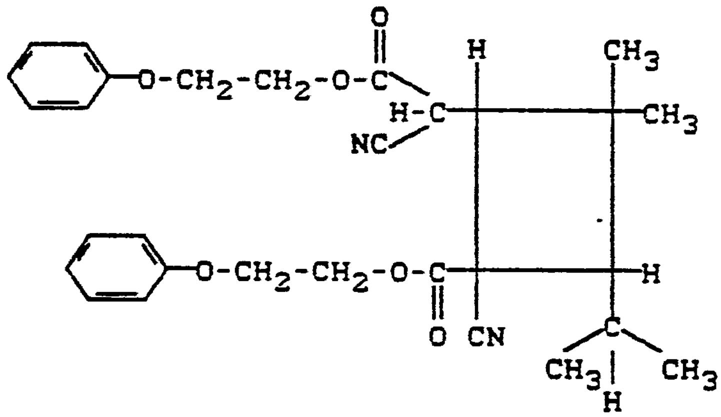

(Ig)

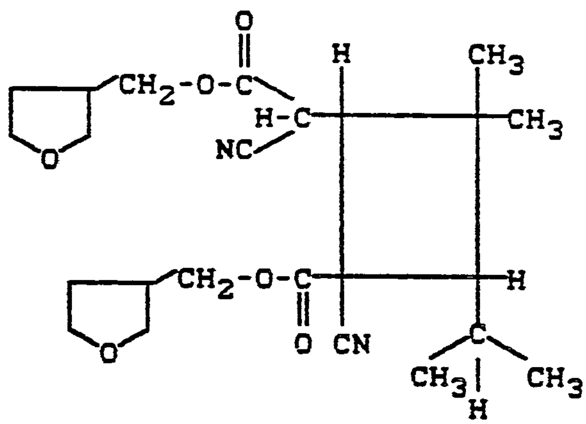

(Ih)

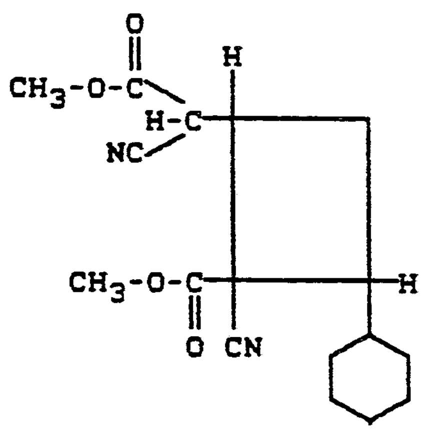

(Ii)

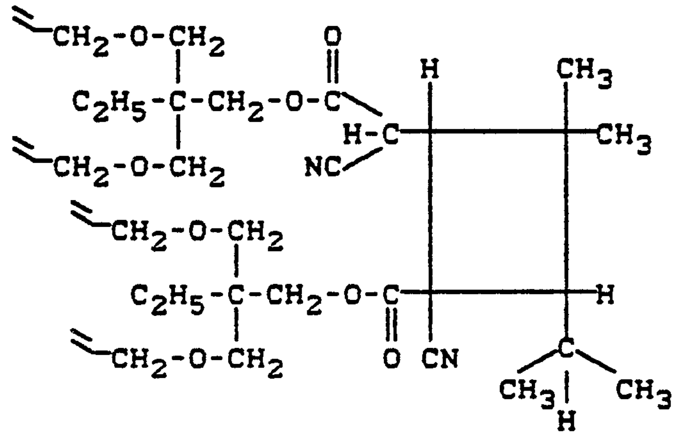

(Ik)

Es wurde weiterhin ein Verfahren zur Herstellung der Cyclobutan-diester-dinitrile der obigen Formel (I) gefunden, das dadurch gekennzeichnet ist, daß man $\beta$-substituierte $\alpha$-Cyanacrylsäureester der Formel

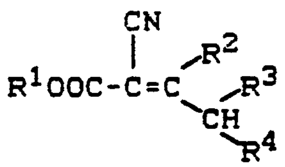

(II),

in welcher $R^1$ -$R^4$ den angegebenen Bedeutungsumfang haben, unter Dimerisierung in Gegenwart einer Base und gegebenenfalls in inerten Lösungsmitteln mit sich selber umsetzt.

Die für das erfindungsgemäße Verfahren erforderlichen Ausgangsstoffe der Formel (II) sind in einer dem Fachmann bekannten Weise durch Knoevenagel-Kondensation aus entsprechend substituerten Cyanessigsäureestern mit Aldehyden oder Ketonen erhalten worden (Organikum, Organisch-chemisches Grundpraktikum (Autorenkollektiv), VEB Deut scher Verlag der Wissenschaften Berlin, 5. Auflage 1965, Seite 444; Weygand-Hilgetag, Organisch-chemische Experimentierkunst, Johann Ambrosius Bart-Verlag Leipzig, 3. Auflage 1964, Seite 923).

Diese Ausgangsstoffe der Formel (II) ergeben in völlig überraschender Weise in Gegenwart katalytischer Mengen von Basen durch eine asymmetrische Dimerisierung ($\alpha$-$\beta$-und $\beta$-$\gamma$-Stellung) Dicyclobutan-diester-dinitrile der Formel (I) gemäß der folgenden Formulierung:

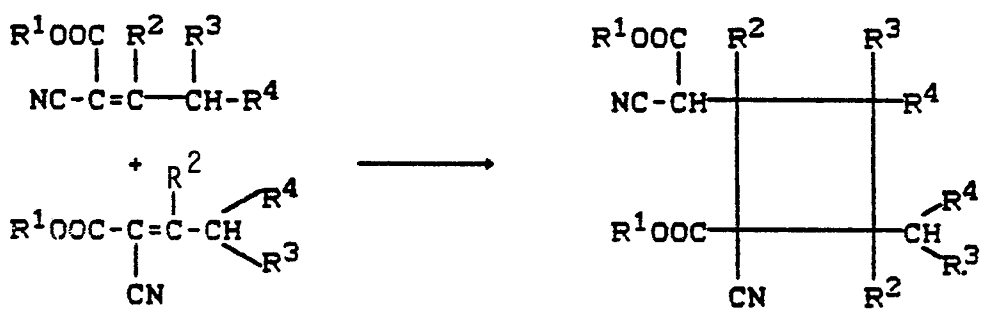

Als Basen für das erfindungsgemäße Verfahren kommen beispielsweise Alkalimetall-oder Erdalkalimetall-Hydroxide oder Alkalimetall-Carbonate, Ammoniak sowie primäre, sekundäre oder tertiäre organische Amine in Frage. In bevorzugter Weise wird in Gegenwart sekundärer oder tertiärer organischer Amine gearbeitet, beispielsweise in Gegenwart von Piperidin, Piperazin, 1,4-Diazabicyclo[2.2.2]-octan, Chinuclidin, Pyridin, Imidazol, 1,2,4-Triazol, Triethylamin, Morpholin, N-Methyl-morpholin, Pyrimidin, Pyrazin, Dibutylamin.

Die genannten Basen werden in einer Menge von beispielsweise 0,01 bis 10 Mol-%, bevorzugt 0,05 bis 5 Mol-%, besonders bevorzugt 0,1 bis 2,5 Mol-%, bezogen auf den Ausgangsstoff der Formel (II) benutzt.

Das erfindungsgemäße Verfahren kann vielfach bei Raumtemperatur durchgeführt werden. Der für das erfindungsgemäße Verfahren zulässige Temperaturbereich ist jedoch größer, beispielsweise 10 bis 100°C.

Das erfindungsgemäße Verfahren kann grundsätzlich ohne Verwendung eines zusätzlichen Lösungsmittels durchgeführt werden. Insbesondere wenn der Ausgangsstoff der Formel (II) bei der gewählten Reaktionstemperatur ein Feststoff ist, wird jedoch mit Vorteil in Gegenwart eines Lösungsmittels gearbeitet. Solche Lösungsmittel müssen unter den Reaktionsbedingungen inert gegenüber den Stoffen der Formel (I) und (II) sein. Als Beispiele seien Alkohole, Ether, Ester, Ketone, aliphatische oder aromatische Kohlenwasserstoffe, aliphatische oder aromatische Chlor-Kohlenwasserstoffe, Nitrobenzol oder N,N-Dialkylsäureamide genannt. Beispiele für solche Lösungsmittel sind:

Alkohole der Formel $R^1OH$, wobei $R^1$ den obengenannten Bedeutungsumfang hat; für den Fall, daß eine Umesterung an den Stoffen (I) und (II) zugelassen wird, kommen jedoch auch Alkohole in Frage, deren Organylrest nicht mit $R^1$ übereinstimmt;

Ether, wie Dioxan, Tetrahydrofuran, Dialkylether; Ketone, wie Aceton oder Methyl-ethyl-keton; Ester, wie Ethylacetat, Butylacetat und andere;

Dimethylformamid, Diethylformamid, Dimethylacetamid; Acetonitril, Dimethylsulfoxid, Benzol, Toluol, Xylol, Hexan, Ligroin, Methylenchlorid, Dichlorethan, Chlorbenzol, Dichlorbenzol.

Die erfindungsgemäßen Cyclobutan-diester-dinitrile sind wertvolle Zwischenprodukte, beispielsweise zum Aufbau weiterer homocyclischer oder heterocyclischer Verbindungen sowie zur Überführung in die den Stoffen der Formel (I) entsprechenden Diamine (durch Hydrierung der Nitrilgruppen), Isocyanate (durch Phosgenierung der durch Hydrierung der Nitrilgruppen entstandenen Amine) und Harnstoffe (durch dem Fachmann bekannte Umsetzung der durch Hydrierung der Nitrilgruppen entstandenen Amine). In bevorzugter Weise können die Stoffe der Formel (I) oder deren genannte Umwandlungsprodukte (besonders die Diamine) jedoch durch Polykondensationsreaktionen in Polymere, wie Polyester, Polyamide oder Polyurethane umgesetzt werden. So kann beispielsweise ein Stoff der Formel (I) durch Polykondensation an seinen beiden Estergruppen, beispielsweise mit Diaminen oder Diolen unter Austritt des Alkohols $R^1OH$ zu Polyestern oder Polyamiden umgesetzt werden. Für diese Polykondensationszwecke werden vor allen Dingen Stoffe der Formel (I) eingesetzt, in denen $R^1$ ein niederer Alkylrest, beispielsweise mit 1 - 4 C-Atomen ist. Durch Polykondensation von Umwandlungsprodukten der Stoffe (I), in denen beispielsweise die beiden Nitrilgruppen zu Aminogruppen hydriert worden sind, kann man durch Umsetzung mit Phosgen zu Polyurethanen kommen. Die Erfindung betrifft daher weiterhin die Verwendung der Cyclobutan-diester-dinitrile der Formel (I) als Ausgangsstoff zur Herstellung von Diaminen, Isocyanaten, Harnstoffen, Cycloharnstoffen und Heterocyclen sowie die Verwendung dieser Cyclobutan-diester-dinitrile als solche oder nach Umwandlung ihrer Nitrilgruppen in Aminogruppen als Monomere zur Herstellung von Polyestern, Polyamiden oder Polyurethanen.

<u>Beispiele</u>

<u>Beispiele 1</u> (β-Isopropyl-α-cyanacrylsäure-methylester)

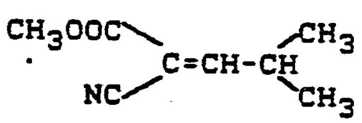

$C_8H_{11}NO_2$ (153)

Aus 72 g Isobutyraldehyd, 500 ml Toluol, 99 g Cyanessigsäuremethylester, 8 g Ammonacetat und 13 g Essigsäure. Kp 50 - 53°C bei 0,05 mbar, Ausbeute 124 g (99,5 %ig lt. gaschromatographischer Analyse).

Beispiel 2

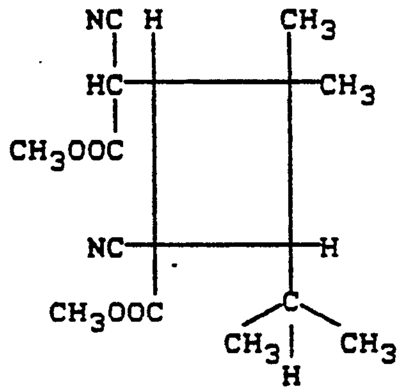

$C_{16}H_{22}N_2O_4$ (304)

90 g der in Beispiel 1 hergestellten Verbindung in 50 ml Methanol wurden unter Rühren mit 0,6 g Piperidin versetzt; man ließ über Nacht stehen und saugte das Kristallisat ab.

Ausbeute 75 g farblose Kristalle, Fp. 120-125°C;
Analyse für $C_{16}H_{22}N_2O_4$ (306)
ber. C: 62.7, H: 7.2, N: 9.15
gef. C: 62.9, H: 7.4, N: 9.20

Größtes Fragment im Massenspektrum $M^+$ bei 306; Molmasse osmometrisch in Aceton: 320; die Ringstruktur wird durch Röntgendiffraktometrie belegt.

Beispiel 3

156 g der in Beispiel 2 hergestellten Verbindung wurden mit 58 g 1,6-Hexamethylendiamin unter Rühren auf 150°C erhitzt und Methanol abdestilliert, wobei die Reaktionstemperatur mit fortlaufender Kondensation laufend bis zu 220°C erhöht werden mußte.

Das zähflüssige Polykondensat wurde bei 220°C ausgegossen, zog verstreckbare Fäden und erstarrte langsam.

Beispiel 4 ($\beta$-Isopropyl-$\alpha$-cyanacrylsäure-n-butylester)

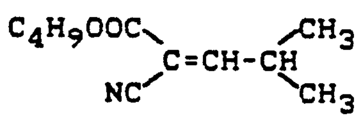

$C_{11}H_{17}NO_2$ (195)

Aus 141 g Cyanessigsäurebutylester, 80 g Isobutyraldehyd, 8 g Ammonacetat und 12 g Essigsäure in 500 ml Toluol am Wasserabscheider; Kp 105°C bei 0,09 mbar, Ausbeute 100 g (99.9 %ig lt. gaschromatographischer Analyse).

Beispiel 5

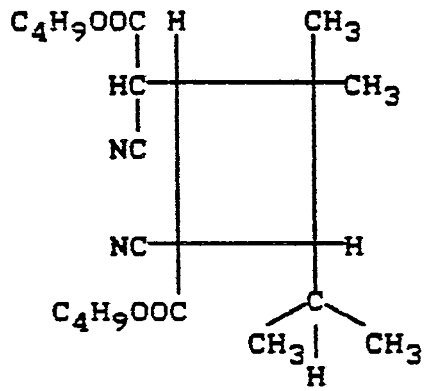

$C_{22}H_{34}N_2O_4$ (390)

95 g der in Beispiel 4 hergestellten Verbindung in 50 ml Butanol und 0,5 g Piperidin ließ man über Nacht stehen, saugte ab, wusch mit wenig Butanol nach und trocknete.
Ausbeute 60 g, Fp 80-82°C;
Analyse für $C_{22}H_{34}N_2O_4$ (390)
ber. C: 67.7, H: 8.7, N: 7.1
gef. C: 67.7, H: 8.4, N: 7.0.

Beispiel 6 (β-Isopropyl-α-cyanacrylsäure-allylester)

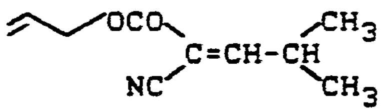

$C_{10}H_{13}NO_2$ (179)

Aus 125 g Cyanessigsäureallylester, 72 g Isobutyraldehyd 12 g Essigsäure und 8 g Ammonacetat in 500 ml Toluol; Kp 54°C bei 0,09 mbar;
Ausbeute 159 g (98,0 %ig lt. gaschromatographischer Analyse).

Beispiel 7

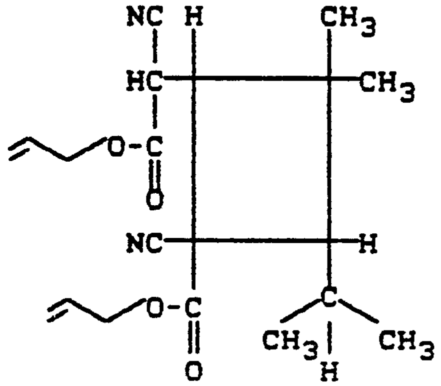

$C_{20}H_{26}N_2O_4$ (358)

50 g der in Beispiel 6 hergestellten Verbindung wurden mit 0,4 g Piperidin versetzt und über Nacht stehen gelassen, mit 10 ml kaltem Methanol aufgerührt und abgesaugt;
Ausbeute 38 g, Fp 79-82°C;
Analyse für $C_{20}H_{26}N_2O_4$(358)
ber. C: 67.0, H: 7.25, N: 7.8
gef. C: 67.1, H: 7.10, N: 7.8.

Beispiel 8

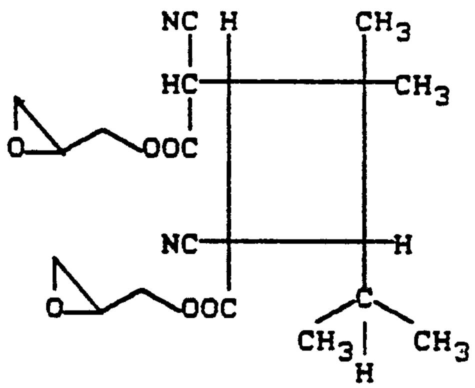

$C_{20}H_{26}N_2O_6$ (390)

17,9 g der in Beispiel 7 hergestellten Verbindung wurden in 25 ml $CHCl_3$ gelöst und bei Raumtemperatur mit 17,2 g m-Chlorperbenzoesäure versetzt und für 3 h auf 40°C erwärmt; es wurde abgekühlt und von der m-Chlorbenzoesäure abgesaugt; die Mutterlauge wurde eingeengt, in Ether aufgekocht und der Rückstand aus Waschbenzin-Toluol (1:1) umkristallisiert.
Ausbeute 8 g, Fp 86-88°C;
Analyse für $C_{20}H_{26}N_2O_6$ (390)
ber. C: 61.5, H: 6.7, N: 7.1
gef. C: 62.3, H: 6.8, N: 7.1

Das Bis-epoxid kann mit Diaminen oder Carbonsäureanhydriden in üblicher Weise zur Epoxidhärtung verwendet werden. 18,5 g der oben formulierten Verbindung und 7,7 g Phthalsäureanhydrid werden bei 135°C homogenisiert und während 15 Stunden bei 150°C in einer Metallform getempert. Die Biegefestigkeit des Gießharzes beträgt 125 MPa (DIN 53 452).

Beispiel 9

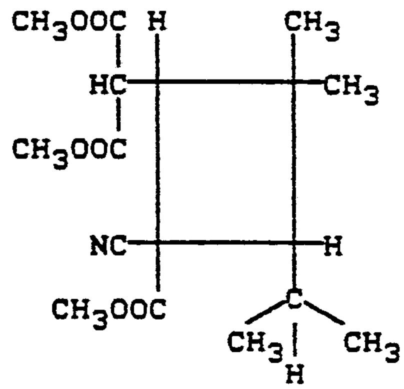

$C_{17}H_{25}NO_6$ (339)

In 200 ml Methanol wurde bei 60°C zur Sättigung HCl eingeleitet; dann wurden 16 g der in Beispiel 2 hergestellten Verbindung zugesetzt und für 5 h auf 60°C erwärmt; es wurde abgekühlt und in 500 g Eis gegossen, gewaschen, getrocknet und aus Waschbenzin umkristallisiert.
Ausbeute 9 g, Fp 95-99°C;
Analyse für $C_{17}H_{25}NO_6$ (339)
ber. C: 60.2, H: 7.3, N: 4.1
gef. C: 60.4, H: 7.1, N: 4.4.

Beispiel 10

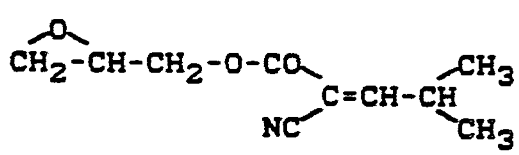

$C_{10}H_{13}NO_3$ (195)

86 g m-Chlorperbenzoesäure wurden in 325 ml Chloroform gelöst und langsam mit 89,5 g β-Isopropyl-α-cyan-acrylsäure-allylester von Beispiel 6 versetzt; anschließend wurde für 20 Stunden auf 70°C erwärmt, abgekühlt und von der m-Chlorbenzoesäure abgetrennt. Der Rückstand wurde eingeengt und destilliert. Ausbeute 52 g; Kp. des Glycidylesters: 96°C bei 0,15 mbar; Reinheit 95 % nach gaschromatographischer Analyse. Die Epoxidbildung wurde durch Kernresonanz-und Massenspektren gesichert.

Beispiel 11

20 g der in Beispiel 10 hergestellten Verbindung wurden mit 0,2 g Piperidin versetzt, über Nacht stehen gelassen und dann in 5 ml kaltem Glycid aufgerührt. Der sich bildende kristalline Niederschlag wurde abgesaugt und aus Waschbenzin-Toluol (1:1) umkristallisiert. Ausbeute 7 g; Fp. 85-87°C; Analyse und Spektren entsprachen den Ergebnissen, die in Beispiel 8 angegeben wurden, und somit der folgenden Struktur:

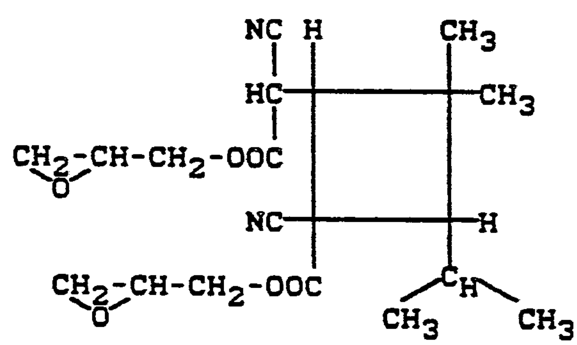

## Ansprüche

1. Cyclobutan-diester-dinitrile der Formel

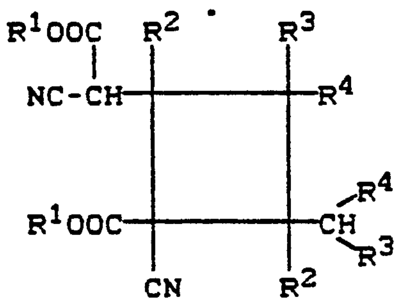

,

in der
$R^1$ für gegebenenfalls substituiertes Alkyl, Alkenyl, Cycloalkyl, Cycloalkenyl, Aryl, Aralkyl, Hetero(cyclo)alkyl oder Heteroaryl steht,
$R^2$, $R^3$ und $R^4$ unabhängig voneinander Wasserstoff oder gegebenenfalls substituiertes Alkyl, Aryl, Aralkyl, Alkoxy, Aryloxy oder Aralkoxy bedeuten und worin weiterhin
$R^2$ und $R^3$ oder $R^3$ und $R^4$ gemeinsam mit den C-Atomen, die sie substituieren, Cycloalkyl oder Cycloalkenyl bilden können, die Sauerstoff-oder Schwefelatome als Ringglieder enthalten können.

2. Cyclobutan-diester-dinitrile nach Anspruch 1, in denen an die Stelle von $R^2$, $R^3$ und $R^4$ die Reste $R^{12}$, $R^{13}$ und $R^{14}$ treten, die unabhängig voneinander Wasserstoff, $C_1$-$C_{12}$-Alkyl, Phenyl oder Benzyl bedeuten.

3. Cyclobutan-diester-dinitrile nach Anspruch 1, in denen an die Stelle von $R^2$, $R^3$ und $R^4$ die Reste $R^{22}$, $R^{23}$ und $R^{24}$ treten, die unabhängig voneinander Wasserstoff oder $C_1$-$C_4$-Alkyl bedeuten.

4. Cyclobutan-diester-dinitrile nach Anspruch 1, in denen an die Stelle von $R^2$, $R^3$ und $R^4$ die Reste $R^{32}$, $R^{33}$ und $R^{34}$ treten, wobei
$R^{32}$ Wasserstoff und
$R^{33}$ und $R^{34}$ unabhängig voneinander $C_1$-$C_4$-Alkyl
bedeuten.

5. Cyclobutan-diester-dinitrile nach Ansprüchen 1 bis 4, in denen an die Stelle von $R^1$ der Rest $R^{11}$ tritt, der $C_1$-$C_{12}$-Alkyl, $C_2$-$C_{12}$-Alkenyl, $C_5$-$C_6$-Cycloalkyl, $C_5$-$C_6$-Cycloalkenyl, Aryl, Aralkyl oder Heterocycloalkyl-(alkyl) bedeutet.

6. Cyclobutan-diester-dinitrile nach Ansprüchen 1 bis 4, in denen anstelle von $R^1$ der Rest $R^{21}$ tritt, der $C_1$-$C_4$-Alkyl, $C_2$-$C_4$-Alkenyl, Phenyl, Benzyl, Phenylethyl oder Glycidyl bedeutet.

7. Verfahren zur Herstellung von Cyclobutan-diester-dinitrilen der Formel

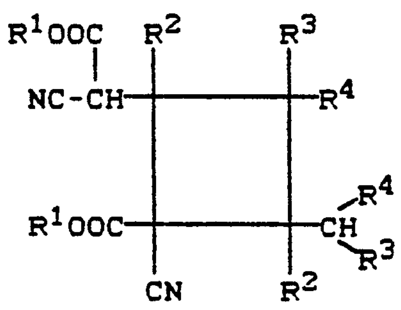

,

in der
$R^1$ für gegebenenfalls substituiertes Alkyl, Alkenyl, Cycloalkyl, Cycloalkenyl, Aryl, Aralkyl, Hetero(cyclo)alkyl oder Heteroaryl steht,
$R^2$, $R^3$ und $R^4$ unabhängig voneinander Wasserstoff oder gegebenenfalls substituiertes Alkyl, Aryl oder Aralkyl bedeuten und worin weiterhin
$R^2$ und $R^3$ oder $R^3$ und $R^4$ gemeinsam mit den C-Atomen, die sie substituieren, Cycloalkyl oder Cycloalkenyl bilden können, die Sauerstoff-oder Schwefelatome als Ringglieder enthalten können,
dadurch gekennzeichnet, daß man $\beta$-substituierte $\alpha$-Cyanacrylsäureester der Formel

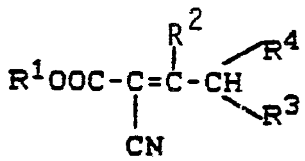

,

in welcher

R¹ bis R⁴ den angegebenen Bedeutungsumfang haben, unter Dimerisierung in Gegenwart einer Base und gegebenenfalls in inerten Lösungsmitteln mit sich selber umsetzt.

8. Verfahren nach Anspruch 7, dadurch gekennzeichnet, daß als Base sekundäre oder tertiäre organische Amine eingesetzt werden.

9. Verwendung der Cyclobutan-diester-dinitrile gemäß Anspruch 1 oder nach Umwandlung der Nitrilgruppen in Aminogruppen als Monomere zur Herstellung von Polyestern, Polyamiden oder Polyurethanen.

10. Verwendung der Cyclobutan-diester-dinitrile nach Ansprüchen 1 bis 6 als Ausgangsstoffe zur Herstellung von Aminen, Isocyanaten, Harnstoffen, Cycloharnstoffen und Heterocyclen.

Europäisches Patentamt

# EUROPÄISCHER RECHERCHENBERICHT

Nummer der Anmeldung

EP 87 10 3797

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 819 677 (D.M. GALE)<br>--- | 1 | C 07 C 121/46<br>C 08 G 18/00<br>C 08 G 63/00<br>C 08 G 69/00<br>C 07 C 87/34<br>C 07 C 119/042<br>C 07 C 127/15 |
| A | US-A-3 642 859 (D.M. GALE)<br>--- | 1 | |
| A | US-A-4 133 961 (W.C. WATKINS)<br>----- | 1 | |
| | | | RECHERCHIERTE SACHGEBIETE (Int. Cl.4) |
| | | | C 07 C 121/46 |

Der vorliegende Recherchenbericht wurde für alle Patentansprüche erstellt.

| Recherchenort | Abschlußdatum der Recherche | Prüfer |
|---|---|---|
| BERLIN | 04-06-1987 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82